# EUROPEAN PATENT SPECIFICATION

(11) **EP 1 743 309 B1**
(45) Date of publication and mention of the grant of the patent: **19.10.2011**
(21) Application number: 04821989.3
(22) Date of filing: 15.04.2004
(51) Int. Cl.: G09B 23/28, A61B 5/00

(54) **SYSTEM FOR INSTRUCTING REMOVAL OF CATARACT TISSUE**
SYSTEM ZUM ANLEITEN DER ENTFERNUNG VON KATARAKTGEWEBE
SYSTEME D'INSTRUCTIONS DESTINE AU RETRAIT DU TISSU DE LA CATARACTE

(43) Date of publication of application: 17.01.2007
(73) Proprietor: Novartis AG, 4056 Basel (CH)
(72) Inventor: MACKOOL, Richard, J., Astoria NY 11103 (US)
(74) Representative: Hanna, Peter William Derek
(86) International application number: PCT/US2004/011672
(87) International publication number: WO 2005/111970

(56) References cited:
- US-A- 4 959 049
- US-A- 4 959 049
- US-A- 5 454 722
- US-A- 6 155 975
- US-A- 6 155 975
- US-A- 6 159 175

## Description

### FIELD OF THE INVENTION

The present invention relates to software for instructing the removal of body tissue, and more particularly relates to a computer program product for instructing the removal of cataract tissue during cataract surgery.

### BACKGROUND OF THE INVENTION

Phacoemulsification is a method of removing cataracts through the use of an ultrasonic needle vibrating at ultrasonic frequencies. The needle vibration breaks up the cataract into pieces which are emulsified and then gently aspirated out of the eye. This method of cataract removal is preferred in that it requires only a tiny incision at the perimeter of the cornea through which the needle is inserted. The surgically induced trauma to the eye and the tissues surrounding the eye caused by phacoemulsification surgery is so minimal that it allows the patient to regain vision relatively quickly after the surgery.

A current phacoemulsification technique for removing or assisting in cataract removal employs a hollow needle, either straight or angulated, which either rotates or oscillates in a to and fro manner. The frequency of oscillation/rotation is controllable by the surgeon, and generally is pre-programmed into the console controlling the phacoemulsification handpiece. The rotation or oscillation of the needle is believed to assist in the removal of tissue, particularly human lens (cataract) tissue. The to and fro oscillation method is commercially available from Alcon Laboratories, Inc., of Fort Worth, Texas, under the trademark Neosonix®. A method in which the tip of the needle rotates completely in one direction or the other (at a rate of approximately 4,000-5,000 rpm), known as "phacotmesis," also is known.

There is a major disadvantage to the to and fro oscillatory method. During cataract removal, it is often desirable to use strong vacuum forces to remove cataract tissue from the eye in order to reduce or eliminate the need to use additional energy, such as ultrasonic vibration. If used to excess, ultrasonic vibrations have been shown to damage ocular tissues such as the corneal endothelium or iris, which line the anterior chamber of the eye in which the ultrasonic needle is inserted. The applied vacuum aspirates the cataract tissue through the shaft of the hollow needle.

When cataract tissue obstructs the orifice of the needle, the vacuum within the needle immediately begins to increase to approximately 600 mm Hg or more in modern cataract removal systems such as the Alcon® Advantec® Legacy®. This increasing vacuum is often successful in causing removal of the tissue. The to and fro oscillatory method, if activated during this period of increased vacuum force, generally results in the elimination of the occlusion by permitting the occluding portion of the cataract to slip off the rotating needle. This can occur with the straight needle design and is even more likely to occur with the angulated needle design because of the relocation of the tip of the needle in space which occurs immediately upon activation of the oscillatory mechanism. Thus, when activated, the oscillatory method results in a loss of the desirable vacuum force upon the cataract tissue.

Current phacoemulsification systems, such as the Alcon® Advantec® Legacy@ with Neosonix®, are used independently of the vacuum and are dependent only on an achieved ultrasonic power application. That is, the needle oscillation can be preset to activate at given percentage of ultrasonic power applied. This phacoemulsification system also permits automated changes in ultrasonic power application as a function of vacuum. For example, when a certain level of vacuum is achieved, ultrasonic power may be preprogrammed to decrease to a set lower level, to be applied intermittently in short pulses, etc. This arrangement exists because it is believed that the achieved vacuum force will assist in the removal process and lower levels/intermittent amounts of ultrasonic energy can thus be used to remove the cataract.

Other systems for removal of cataract tissue have been disclosed in United States Patent Nos. 5,019,036; 5,106,367; 5,213,569; 5,403,323; 5,634,920; 5,788,679; 5,830,176; 6,042,566; 6,258,053 and 6,428,508. These patents disclose and claim devices for use in phacoemulsification with which the present invention may be utilized.

It would be desirable to configure software within the controlling console controlling the cataract removal process such that the activation of the to and fro oscillatory mechanism and/or the ultrasonic power may be preset to occur automatically once a preset vacuum level is achieved, whether as an absolute vacuum level or as percentage of maximum vacuum the console will permit during the process. Linking the activation of the to and fro needle oscillation or application of ultrasonic power to achieving a threshold vacuum level permits sequential application of vacuum and oscillatory or ultrasonic forces to the cataract tissue to be removed. The achieved vacuum also would reduce the likelihood of cataract tissue escaping from the needle tip during oscillation or ultrasonic vibration. If the applied vacuum successfully removed the cataract tissue before the threshold vacuum level is achieved, activation of the needle oscillation or ultrasonic vibration would be unnecessary.

Heretofore, we are unaware of any phacoemulsification system in which the to and fro needle oscillation is improved by avoiding activation thereof until a pre-determined level of vacuum has been achieved. We also are unaware of any phacoemulsification system in which the application of ultrasonic power is improved by avoiding activation thereof until a predetermined level of vacuum has been achieved.

### SUMMARY OF THE INVENTION

A system as disclosed in claim 1.

In a preferred embodiment, the software routine is configured to send further instructions to terminate the oscillation of the needle tip in response to, the vacuum monitoring signal indicating that the magnitude of said vacuum force has decreased below threshold. In a further preferred embodiment, the software routine is responsive to a user-initiated oscillation termination signal to send further instructions to terminate the oscillation of the tip. In this embodiment, the software routine avoids sending instructions to terminate the to and fro oscillation of the tip unless the user- initiated oscillation termination signal is received.

The software routine also is configured to send further instructions to effect alteration of the oscillation of the tip in response to the vacuum monitoring signal indicating that the magnitude of the vacuum force within the needle shaft has decreased below threshold level.

The system of the present invention also comprises a software routine that generates a vacuum generating signal indicative of the vacuum force to be applied within the shaft of a phacoemulsification needle. The software routine is responsive to a vacuum monitoring signal that indicates that the magnitude of vacuum force within the needle shaft has reached a threshold level as a result of partial or complete occlusion of the tip of the needle by the cataract tissue, so as to send instructions to activate the application of ultrasonic power via the needle tip.

In a preferred embodiment, the software routine is configured to send further instructions to terminate the application of ultrasonic power in response to the vacuum monitoring signal indicating that the magnitude of said vacuum force has decreased below threshold. In a further preferred embodiment, the software routine is responsive to a user- initiated ultrasonic termination signal to send further instructions to terminate the application of ultrasonic power via the tip. In this embodiment, the software routine avoids sending instructions to terminate the application of ultrasonic power unless the user-initiated ultrasonic termination signal is received.

The software routine also is configured to send further instructions to effect alteration of the ultrasonic power in response to the vacuum monitoring signal indicating that the magnitude of the vacuum force within the needle shaft has decreased below threshold level.

A method of instructing removal of cataract tissue, which is not claimed herein, may comprise: (a) generating a vacuum generating signal indicative of the vacuum force to be applied within the hollow shaft of a phacoemulsification needle; and (b) sending instructions to activate the to and fro oscillation of the needle tip in response to a vacuum monitoring signal indicating that the vacuum force has reached a threshold level as a result of partial or complete occlusion of the needle tip by the cataract tissue.

A method of instructing the removal of cataract tissue, which is not claimed herein, may comprise: (a) generating a vacuum generating signal indicative of the vacuum force to be applied within the hollow shaft of a phacoemulsification needle; and (b) sending instructions to activate the application of ultrasonic power at the needle tip in response to a vacuum monitoring signal indicating that the vacuum force has reached a threshold level as a result of partial or complete occlusion of the needle tip by the cataract tissue.

### BRIEF DESCRIPTION OF THE DRAWINGS

Fig. 1 is a flow chart of a software routine suited for use as part of a cataract removal system in accordance with the present invention.

Fig. 2 is a flow chart of a further software routine suited for use as part of a cataract removal system in accordance with the present invention.

Fig. 3 is a flow chart of a software routine suited for use as part of another cataract removal system in accordance with the present invention.

Fig. 4 is a flow chart of a further software routine suited for use as part of another cataract removal system in accordance with the present invention.

Fig. 5 is a schematic diagram illustrating the cataract removal system of Figs. 1 & 2.

Fig. 6 is a schematic diagram illustrating the cataract removal system of Figs. 3 & 4.

### DETAILED DESCRIPTION OF THE INVENTION

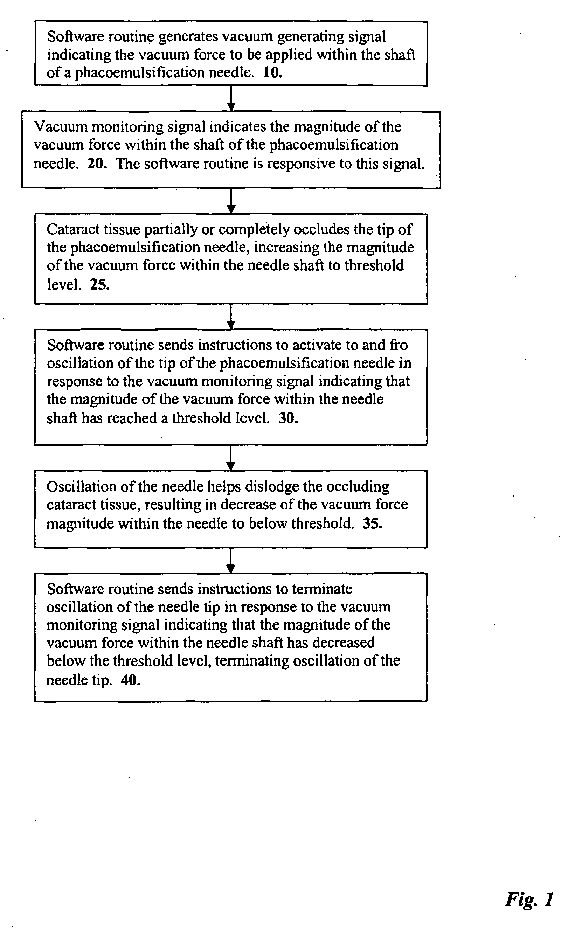

Turning to the drawings, Figs 1-6 represent systems for instructing the removal of cataract tissue. A software routine generates a vacuum generating signal indicating the vacuum force to be applied within the hollow shaft of a phacoemulsification needle. The software routine is responsive to a vacuum monitoring signal that indicates that the magnitude of vacuum force within the needle shaft has reached a threshold level as a result of partial or complete occlusion of the tip of the needle by the cataract tissue, so as to send instructions to activate to and fro oscillation of the needle tip.

Alternatively, the software routine sends instructions to activate the application of ultrasonic power to the needle tip, in response to the vacuum monitoring signal indicating that the magnitude of the vacuum force has reached the threshold level.

Prior to activating the to and fro oscillation of the needle tip or application of ultrasonic power thereto, the cataract tissue is removed solely by the vacuum force applied via the hollow shaft of the phacoemulsification needle, as a result of the vacuum generating signal. Once the oscillation or application of ultrasonic power has been activated, this oscillation/ultrasonic power helps to remove the cataract tissue occluding the needle tip and thus the hollow shaft.

As shown in the embodiment of Fig. 1, the software routine may be configured to send further instructions to terminate the oscillation of the needle tip in response to the vacuum monitoring signal indicating that the magnitude of said vacuum force has decreased below threshold.

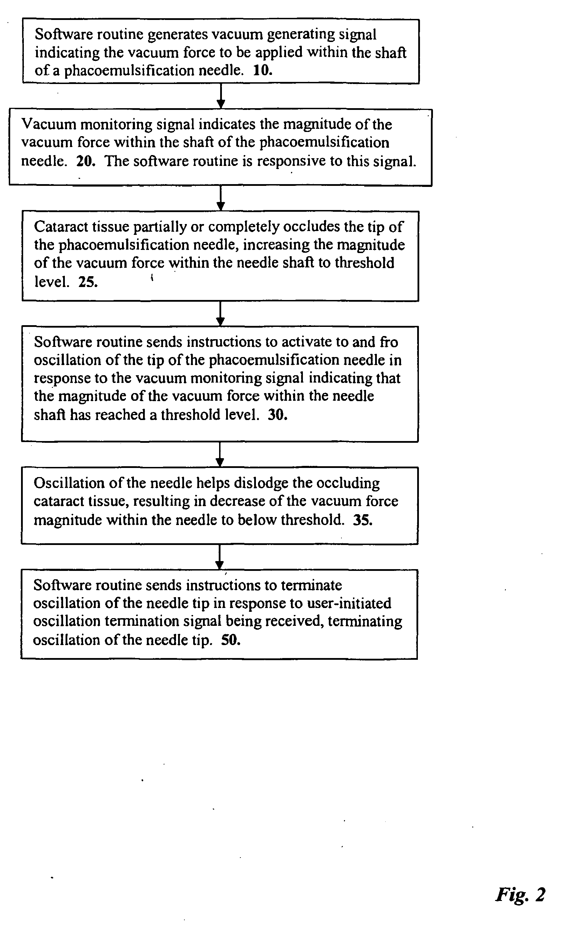

As shown in the embodiment of Fig. 2, and unlike the embodiment of Fig. 1, the software routine alternatively may be responsive to a user-initiated oscillation termination signal to send further instructions to terminate the oscillation of the tip. In this embodiment, the software routine avoids sending instructions to terminate the to and fro oscillation of said tip unless the user-initiated oscillation termination signal is received.

The software routine may also be configured to send further instructions to effect alteration of the oscillation of the needle tip in response to the vacuum monitoring signal indicating that the magnitude of the vacuum force within the needle shaft has decreased below threshold level. Such alterations preferably include speed of needle oscillation, duration of needle oscillation, frequency of pulsing of oscillation, and/or duration of pulses of oscillation.

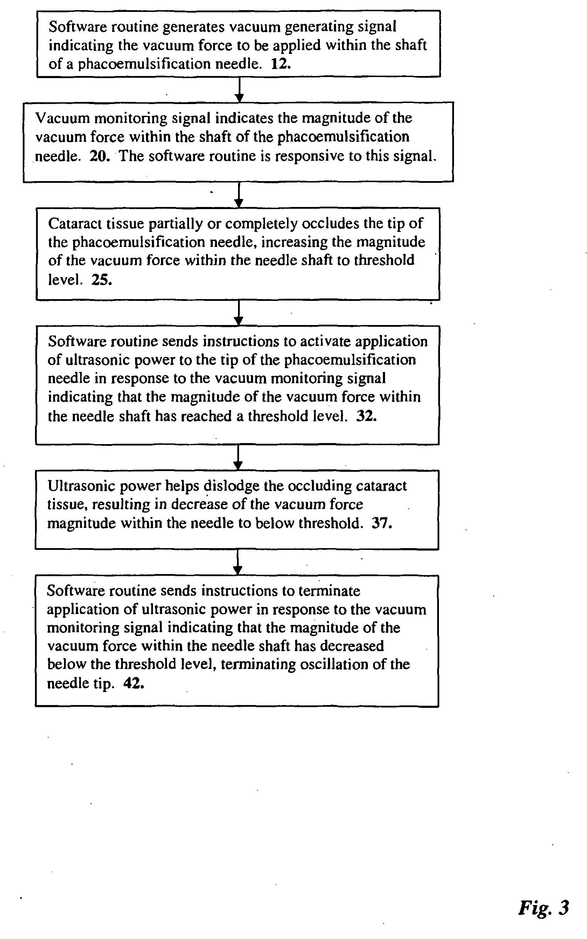

As shown in the embodiment of Fig. 3, the software routine alternatively may be configured to send further instructions to terminate the application of ultrasonic power to the needle tip in response to the vacuum monitoring signal indicating that the magnitude of said vacuum force has decreased below threshold.

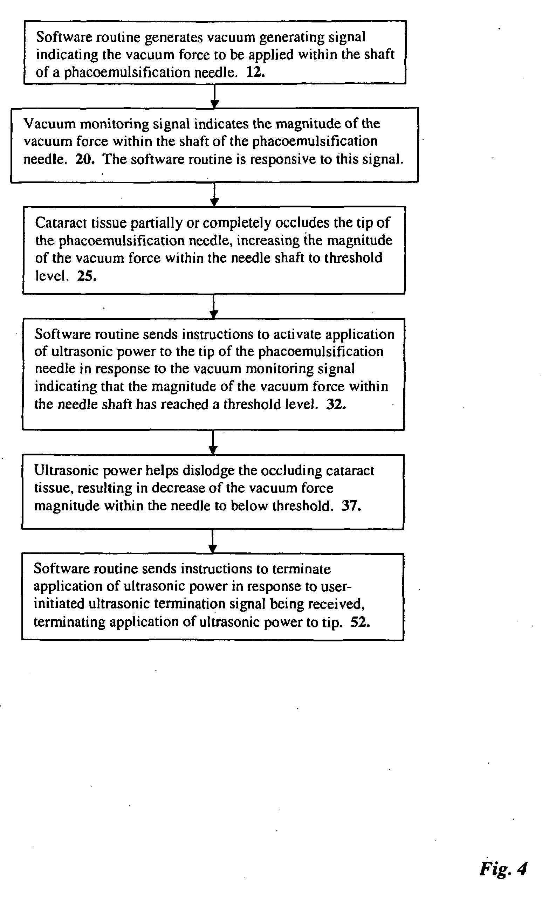

As shown in the embodiment of Fig. 4, and unlike the embodiment of Fig. 3, the software routine further alternatively may be responsive to a user-initiated ultrasonic termination signal to send further instructions to terminate the application of ultrasonic power. In this embodiment, the software routine avoids sending instructions to terminate the application of ultrasonic power unless the user-initiated ultrasonic termination signal is received.

In all of the foregoing embodiments, a vacuum force is maintained within the hollow shaft of the phacoemulsification needle during the to and fro oscillation, to prevent the cataract tissue that is occluding the needle tip, and thus preventing aspiration through the needle shaft, from falling off the tip during oscillation. Rather, as a consequence of this maintained vacuum force, the occluding cataract tissue dislodged from the needle tip by the to and fro oscillation or application of ultrasonic power is immediately aspirated through the hollow shaft of the phacoemulsification needle, rather than falling back to the eye surface, in which case additional vacuum force application will be required to remove the tissue. This maintained vacuum force during needle oscillation/ultrasonic power application renders the system of the present invention superior to conventional phacoemulsification systems employing needle oscillation or ultrasonic power in connection with vacuum force.

The threshold vacuum level for sending instructions to activate the needle oscillation or the application of ultrasonic power, may be pre-set by the surgeon. The threshold vacuum level may be an absolute vacuum level, that is, a selected number of mm Hg. Alternatively, the threshold vacuum level may be pre-set as some percentage of the maximum vacuum allowed during the procedure by the controlling console.

The system of the present invention may be used in connection with any conventional console for controlling the to and fro oscillation of a phacoemulsification needle and/or application of ultrasonic power thereto, and the vacuum force present in the hollow shaft thereof. The computer program products may as well be used with any conventional phacoemulsification handpiece and needle, including angulated and non-angulated needles.

Turning again to Fig. 1, a sequence of functions 10,20, 30 and 40 is performed by the software routine as indicated. The condition 25 as indicated arises before function 30 is carried out. Also, the condition 35 arises before function 40 is carried out. With respect to Fig. 2, the same applies as in Fig. 1, except that function 40 is replaced by function 50.

Turning again to Fig. 3, a sequence of functions 12,20, 32 and 42 is performed by the software routine as indicated. The condition 25 as indicated arises before function 32 is carried out. Also, the condition 37 arises before function 42 is carried out. With respect to Fig. 4, the same applies as in Fig. 3, except that function 42 is replaced by function 52.

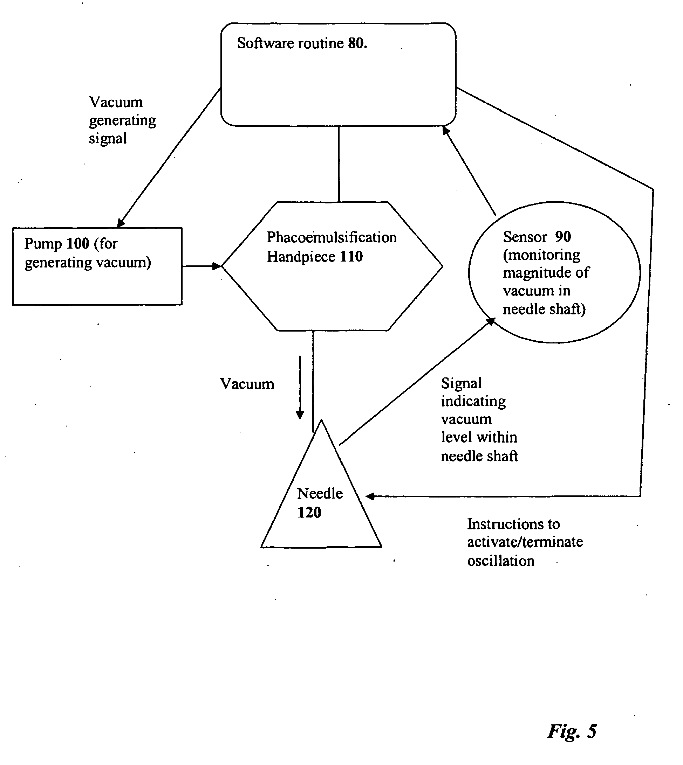

Fig. 5 shows, in a schematic fashion, the interrelationship of the software routine 80, the sensor 90 that senses the vacuum force level within the shaft of the phacoemulsification needle and generates the vacuum monitoring signal, the peristaltic pump 100 that generates the vacuum force within the needle shaft, the phacoemulsification handpiece 110, and the needle 120.

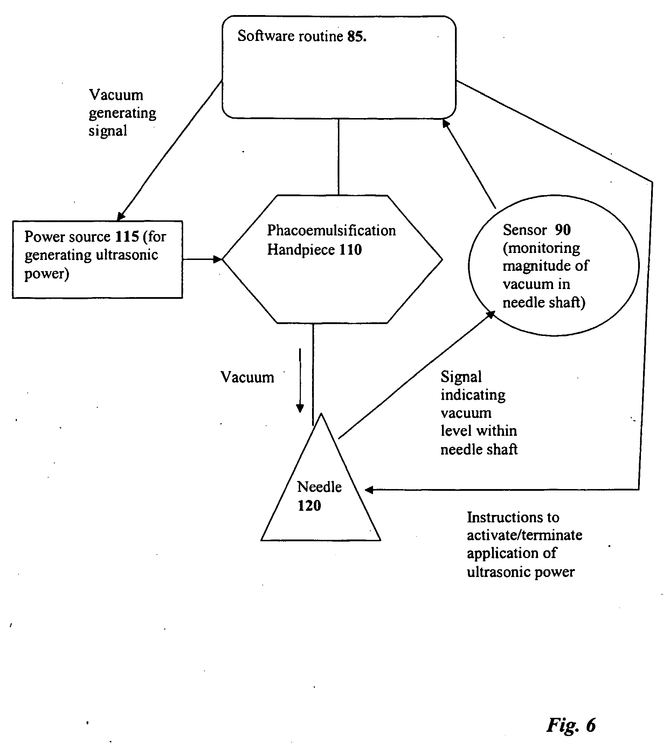

Fig. 6 shows, in a schematic fashion, the alternative interrelationship of the software routine 85, the sensor 90 that senses the vacuum force level within the shaft of the phacoemulsification needle and generates the vacuum monitoring signal, the power source 115 that generates the ultrasonic power, the phacoemulsification handpiece 110, and the needle 120.

## Claims

1. A system for instructing removal of cataract tissue, comprising:
a phacoemulsification needle (120) having a hollow shaft,
means to vibrate the needle ultrasonically,
means (100) to apply a vacuum force to the hollow shaft,
**characterized by**,
a software routine that controls a pump (100) so that said pump generates a vacuum generating signal (80) indicative of a vacuum force to be applied within the hollow shaft of the phacoemulsification needle (120), the software routine being responsive to a vacuum monitoring signal generated by a sensor (90) that indicates that a magnitude of said vacuum force within said hollow shaft has reached a threshold level (20) as a result of partial or complete occlusion of a tip of said phacoemulsification needle by the cataract tissue,
so as to send instructions (30) to activate to and fro oscillation of said tip of said phacoemulsification needle, or
so as to send instructions (32) to activate application of ultrasonic power to said tip of said phacoemulsification needle,
for dislodging or removing (35,37) said occlusion of said tip.

2. The system of claim 1, wherein said software routine is configured to send further instructions (40) to terminate said to and fro oscillation of said tip in response to said vacuum monitoring signal indicating that said magnitude of said vacuum force has decreased below said threshold level.

3. The system of claim 1, wherein said software routine is responsive to a user-initiated oscillation termination signal to send further instructions (50) to terminate said to and fro oscillation of said tip.

4. The system of claim 3, wherein said software routine avoids sending the further instructions (50) to terminate said to and fro oscillation of said tip unless said user-initiated oscillation termination signal is received.

5. The system of claim 1, wherein said software routine is configured to send further instructions to effect alteration of the oscillation of said tip in response to said vacuum monitoring signal indicating that said magnitude of said vacuum force has decreased below threshold level, said alteration being a parameter selected from a group consisting of speed, duration, frequency of pulsing of oscillation, and duration of pulses of oscillation.

6. The system of claim 1, wherein said software routine is configured to send further instructions (42) to terminate said application of ultrasonic power in response to said vacuum monitoring signal indicating that said magnitude of said vacuum force has decreased below said threshold level.

7. The system of claim 1, wherein said software routine is responsive to a user-initiated ultrasonic termination signal to send further instructions (52) to terminate said application of ultrasonic power.

8. The system of claim 7, wherein said software routine avoids sending the further instructions (52) to terminate said application of ultrasonic power unless said user-initiated ultrasonic termination signal is received.

## Patentansprüche

1. System zum Anweisen des Entfernens von Kataraktgewebe, das Folgendes enthält:
eine Phakoemulsifikationsnadel (120), die einen hohlen Schaft aufweist,
Mittel zum Vibrieren der Nadel im Ultraschallbereich,
Mittel (100) zum Anwenden einer Unterdruckkraft auf den hohlen Schaft,
**gekennzeichnet durch**
eine Softwareroutine, die eine Pumpe (100) steuert, sodass die Pumpe ein Unterdruckerzeugungssignal (80) erzeugt, das für eine Unterdruckkraft bezeichnend ist, die innerhalb des hohlen Schaftes der Phakoemulsifikationsnadel (120) anzuwenden ist, wobei die Softwareroutine auf ein Unterdrucküberwachungssignal reagierend ist, das **durch** einen Sensor (90) erzeugt wird, der anzeigt, dass ein Betrag der Unterdruckkraft innerhalb des hohlen Schaftes als Folge eines teilweisen oder vollständigen Verschlusses einer Spitze der Phakoemulsifikationsnadel **durch** das Kataraktgewebe einen Schwellwert (20) erreicht hat,
um so Anweisungen (30) zu senden, um Hin-und-her-Schwingung der Spitze der Phakoemulsifikationsnadel zu aktivieren, oder
um so Anweisungen (32) zu senden, um Anwenden von Ultraschallleistung auf die Spitze der Phakoemulsifikationsnadel zu aktivieren,
um den Verschluss der Spitze zu beseitigen oder zu entfernen (35, 37).

2. System nach Anspruch 1, wobei die Softwareroutine konfiguriert ist, weitere Anweisungen (40) zu senden, um die Hin-und-her-Schwingung der Spitze in Reaktion auf das Unterdrucküberwachungssignal zu beenden, das anzeigt, dass der Betrag der Unterdruckkraft unter den Schwellwert gefallen ist.

3. System nach Anspruch 1, wobei die Softwareroutine auf ein benutzerausgelöstes Schwingungsbeendigungssignal reagierend ist, um weitere Anweisungen (50) zu senden, um die Hin-und-her-Schwingung der Spitze zu beenden.

4. System nach Anspruch 3, wobei die Softwareroutine das Senden der weiteren Anweisungen (50), um die Hin-und-her-Schwingung der Spitze zu beenden, vermeidet, es sei denn, das benutzerausgelöste Schwingungsbeendigungssignal wird empfangen.

5. System nach Anspruch 1, wobei die Softwareroutine konfiguriert ist, weitere Anweisungen zu senden, um Änderung der Schwingung der Spitze in Reaktion auf das Unterdrucküberwachungssignal zu bewirken, das anzeigt, dass der Betrag der Unterdruckkraft unter den Schwellwert gefallen ist, wobei die Änderung ein Parameter ist, der aus einer Gruppe ausgewählt ist, die aus Geschwindigkeit, Dauer, Pulsfrequenz der Schwingung und Dauer der Pulse der Schwingung besteht.

6. System nach Anspruch 1, wobei die Softwareroutine konfiguriert ist, weitere Anweisungen (42) zu senden, um die Anwendung von Ultraschallleistung in Reaktion auf das Unterdrucküberwachungssignal zu beenden, das anzeigt, dass der Betrag der Unterdruckkraft unter den Schwellwert gefallen ist.

7. System nach Anspruch 1, wobei die Softwareroutine auf ein benutzerausgelöstes Ultraschallbeendigungssignal reagierend ist, um weitere Anweisungen (52) zu senden, um die Anwendung von Ultraschallleistung zu beenden.

8. System nach Anspruch 7, wobei die Softwareroutine das Senden der weiteren Anweisungen (52), um die Anwendung von Ultraschallleistung zu beenden, vermeidet, es sei denn, das benutzerausgelöste Schwingungsbeendigungssignal wird empfangen.

## Revendications

1. Système pour demander le retrait d'un tissu de cataracte, comprenant :
une aiguille de phacoémulsification (120) comportant une tige creuse,
des moyens pour faire vibrer l'aiguille de manière ultrasonique,
des moyens (100) pour appliquer une force d'aspiration à la tige creuse,
**caractérisé par**
un sous-programme logiciel qui commande une pompe (100) de sorte que ladite pompe génère un signal de génération d'aspiration (80) indicatif d'une force d'aspiration à appliquer dans la tige creuse de l'aiguille de phacoémulsification (120), le sous-programme logiciel réagissant à un signal de surveillance d'aspiration généré par un capteur (90) qui indique qu'une amplitude de ladite force d'aspiration dans ladite tige creuse a atteint un niveau de seuil (20) en conséquence de l'occlusion partielle ou complète d'une extrémité de ladite aiguille de phacoémulsification par le tissu de cataracte,
de manière à envoyer des instructions (30) pour activer l'oscillation en va et vient de ladite extrémité de ladite aiguille de phacoémulsification, ou
de manière à envoyer des instructions (32) pour activer l'application d'une puissance ultrasonore à ladite extrémité de ladite aiguille de phacoémulsification,
pour déloger ou retirer (35, 37) ladite occlusion de ladite extrémité.

2. Système selon la revendication 1, dans lequel ledit sous-programme logiciel est configuré pour envoyer d'autres instructions (40) pour mettre fin à ladite oscillation en va et vient de ladite extrémité en réponse audit signal de surveillance d'aspiration indiquant que ladite amplitude de ladite force d'aspiration a diminué au-dessous dudit niveau de seuil.

3. Système selon la revendication 1, dans lequel ledit sous-programme logiciel réagit à un signal de fin d'oscillation lancé par l'utilisateur pour envoyer d'autres instructions (50) pour mettre fin à l'oscillation en va et vient de ladite extrémité.

4. Système selon la revendication 3, dans lequel ledit sous-programme logiciel évite d'envoyer les autres instructions (50) pour mettre fin à l'oscillation en va et vient de ladite extrémité à moins que ledit signal de fin d'oscillation lancé par l'utilisateur ne soit reçu.

5. Système selon la revendication 1, dans lequel ledit sous-programme logiciel est configuré pour envoyer d'autres instructions pour effectuer la modification de l'oscillation de ladite extrémité en réponse audit signal de surveillance d'aspiration indiquant que ladite amplitude de ladite force d'aspiration a diminué au-dessous d'un niveau de seuil, ladite modification étant un paramètre sélectionné dans un groupe consistant en une vitesse, une durée, une fréquence d'impulsion d'oscillation et une durée des impulsions d'oscillation.

6. Système selon la revendication 1, dans lequel ledit sous-programme logiciel est configuré pour envoyer d'autres instructions (42) pour mettre fin à ladite application de puissance ultrasonore en réponse audit signal de surveillance d'aspiration indiquant que ladite amplitude de ladite force d'aspiration a diminué au-dessous dudit niveau de seuil.

7. Système selon la revendication 1, dans lequel ledit sous-programme logiciel réagit à un signal de fin de puissance ultrasonore lancé par l'utilisateur pour envoyer d'autres instructions (52) pour mettre fin à ladite application de puissance ultrasonore.

8. Système selon la revendication 7, dans lequel ledit sous-programme logiciel évite d'envoyer les autres instructions (52) pour mettre fin à l'application de puissance ultrasonore à moins que ledit signal de fin de puissance ultrasonore lancé par l'utilisateur ne soit reçu.
